# EUROPEAN PATENT APPLICATION

(11) **EP 1 839 503 A1**
(43) Date of publication of application: **03.10.2007**
(21) Application number: 07104795.5
(22) Date of filing: 23.03.2007
(51) Int. Cl.: A23L 1/304, A23L 2/02, A23L 2/68

(54) **Acid sensation increasing agent, method for increasing the acid sensation, and fruit juice containing beverages having therein the acid sensation increasing agent**

(30) Priority: 24.03.2006 NL 1031439
(71) Applicant: Friesland Brands B.V., 7943 PE Meppel (NL)
(72) Inventor: Dekker, Petra, 6721 LB, Bennekom (NL); Beernink, Roy, 7451 VB, Holten (NL); Geurts, Johannes Marie Wilhelmus, 7559 NK, Hengelo (NL); Rijks, Wilfred, 7213 TC, Gorssel (NL); Bongers, Cornelis Margaretha Theodorus Maria, 5707 KR, Helmond (NL)
(74) Representative: van Loon, C.J.J.

(57) **Abstract**

The invention relates to an acid sensation increasing agent. An acid sensation increasing agent is a substance or composition providing a food and preferably a beverage with a taste which is experienced as being more acid than would be expected on the basis of the pH. The invention further relates to a method for increasing the acid sensation, wherein the acid sensation increasing agent is used. Finally, the invention relates to fruit juice containing beverages and particularly fruit juice having therein the acid sensation increasing agent according to the invention.

## Description

The invention relates to an acid sensation increasing agent. The invention further relates to a method for increasing the acid sensation, in which the acid sensation increasing agent is used. In addition, the present invention relates to fruit juice containing beverages having therein the acid sensation increasing agent, an in particular a fruit juice having an acid sensation which is normally only obtained with a fruit juice having a lower pH.

In this specification and the appended claims, an acid sensation increasing agent is a substance or composition providing a food and preferably a beverage, such as a soft drink, with a taste (at least a taste effect) which is experienced as being more acid than would be expected on the basis of the pH. In other words, an acid sensation increasing agent ensures that a product, in which it is included, provides an acid perception, without an associated (further) decrease of the pH. Just like a sweetener provides a sweet perception, the acid sensation increasing agent according to the invention to be described below is an "acidifier".

More in general, the present invention is in the field of compositions for oral use, and in particular in the field of foods and beverages, although the invention is also applicable for other compositions for oral use such as dental care agents.

When, in these compositions for oral use, the taste attribute "fresh" is desired, usually acid compounds are added. These acid compounds result in a decrease of the pH. When the pH of the oral compositions decreases below a pH lower than 5.5 and particularly with a pH of lower than 3-3.5, adverse effects may occur for the teeth. With acid pH, the dental enamel becomes softer and dental erosion may occur. Depending on the composition of the orally applied composition, calcium ions may be leached out with a pH below the so-called dental erosion potential.

Dental erosion can be defined as "pathological, chronic, local, painless loss of hard dental tissue which is chemically etched away from the tooth surface by acid and/or chelation without intervention of bacteria".

More precisely, acid in orally applicable compositions can result in dental erosion, where the dental enamel softens and slowly dissolves. Different factors in the mouth, such as mouth locomotion, saliva quality and eating and drinking behavior, and in the composition itself, such as pH, type of acid present, buffer capacity and calcium phosphate balance of the composition influence the actual erosive action. Saliva can stop an acid attack on the teeth by neutralizing the acid from the food and particularly from a beverage. How quickly the saliva can do this varies by orally applicable composition and particularly depends on the pH, the buffer capacity and the calcium phosphate balance of the composition. The lower the buffer capacity, the more quickly the acid attack on the teeth can be stopped. The lower the pH, the greater the risk of erosion is. The better the calcium phosphate balance, the fewer calcium ions from the teeth pass into dissolution.

In the last few years, much research has been done to deal with the increasingly large problem of dental erosion.

In part, the approach of this research was to increase the calcium (ion) content in compositions to be applied orally. An increased calcium (ion) content does not only contribute to less dissolution of calcium from the teeth, but, also, many calcium forms are usable to increase the pH of a product. However, an increased calcium concentration entails an number of problems. Thus, there are taste problems with too high a calcium concentration, because not all calcium salts or compounds are present in solution, which may, for instance, cause a "rough" mouthfeel. In addition, the use of calcium compounds fairly often results in turbidity, which is undesired with, for instance, a soft drink.

Therefore, WO-A-92/05711 proposes a method for preventing dental erosion by adding 0.02-0.15 wt.% calcium in the form of a calcium citrate maleate complex to acid beverages. The citrate maleate complex helps to increase the solubility of the calcium so that calcium-enriched beverages can be obtained without precipitation of calcium compounds occurring.

In European patent application 1 382 263, it is described that an effective reduction of dental erosion in acid oral compositions can be obtained by setting the amounts of calcium and acidulant such that the molar ratio of calcium:acidulant is between 0.3 and 0.8 and the pH of the composition is between 3.5 and 4.5.

As a calcium source, any suitable calcium salt may be used, calcium carbonate, calcium hydroxide, calcium citrate, calcium maleate, calcium lactate, calcium chloride, calcium glycerophosphate and calcium formate being mentioned by name.

Because of the large increase of erosion damage to the teeth in particularly the western world (60% of the people under 20 have some degree of dental damage due to erosion), the present inventors have also put much effort into research into possible dental erosion-limiting compositions and particularly fruit juices. This research was primarily aimed at reducing the dental erosion potential of existing products while maintaining the taste and other properties of those products, at least without causing large sensory differences.

In Table 1, the pH and buffer capacity are shown of a number of beverages. Although it is known that the factors acid type and concentration, pH and buffer capacity all play a role in the risk of dental erosion, it is not clear which factor plays the leading role. So, on the basis of these data, it is difficult to predict for every beverage to what extent there is a risk of dental erosion. For instance, in table 1, it cannot be said which beverage scores more positively with regard to erosive action: Coca Cola® or Fanta Orange®. Cola has a lower pH, but also a lower buffer capacity. The only thing which can theoretically be assumed is that, when the pH is higher, the buffer capacity is lower and that, when the calcium concentration of beverage A is higher compared to a beverage B, then beverage A is less erosive than beverage B. Thus, milk is less erosive compared to orange juice.

**Table 1. pH and buffer capacity of different beverages**

| **Beverage** | **pH** | **Buffer capacity (mmole OH/l) to pH 5.5** | **Buffer capacity (mmole OH/l) to pH 7** |
|---|---|---|---|
| Coca Cola® | 2.4 | 9 | 25 |
| Fanta Orange® | 2.9 | 32 | 51 |
| Orange juice | 3.8 | 78 | 107 |
| Milk | 6.6 | 0 | 0.5 |

Of the very large group of compounds tested for use in orally applicable compositions, the inventors have found that lactate ions show a special effect. The lactate ions have an acid sensation increasing taste effect.

Whereas, for instance, calcium phosphate has a better action to prevent dental erosion, this does not provide a "more acid" perceiving effect. Tricalcium phosphate is found to provide the product in which it is used with a sweeter, rounder taste. In addition, calcium phosphate causes turbidity.

Lactate ions, however, make the product to which they are added taste more acid, without the pH being reduced proportionally. In other words, trained taste panel members experience the taste of orally applicable products as having a lower pH than the measured pH.

Because the presence of calcium ions has a proven positive effect on preventing dental erosion, the lactate is most preferably used in the form of one or more calcium and lactate-containing compounds and preferably in the form of calcium lactate. A commercially available calcium lactate that can suitably be used for the invention is Puracal-PP FCC.

By adding a lactate ion source and particularly calcium lactate to a composition for oral application, according to the invention, it is possible to prepare a composition with an acid taste sensation, or a more "fresh" taste without the pH of that composition getting a more erosive value for this. That means that the use of a lactate ion source, and particularly calcium lactate, enables generation of more acid tasting products without the pH dropping below the dental erosion potential.

Therefore the present invention also comprises the use of a lactate ion source in a composition for oral application, for instance in a food and preferably in a beverage and particularly in a fruit juice.

In a further aspect, the invention relates to a method for increasing the acid sensation of an orally applicable composition comprising the addition of an effective amount of lactate ions. Preferably, the lactate ions are added in combination with calcium ions, and most preferably in the form of calcium lactate.

The orally applicable composition to which the lactate ions are added is preferably a food, such as a beverage and most preferably a fruit juice. However, the use in oral care products, such as mouthwash products and dental care products, is not excluded.

In a preferred embodiment of the method, the pH of the composition obtained is, after addition of the lactate ions, between 3 and 5.5, preferably between 3 and 3.5. In this specification and the appended claims, the pH is understood to mean the measured pH, and more specifically the pH as measured on the composition (as such, i.e. undiluted) using a WTW pH 325 pH meter, which has been calibrated with two buffer solutions of pH=4.01 and pH=6.98, respectively, at a temperature of 25°C.

The increase of the acid sensation can be quantified by a trained taste panel (member). Studies by the present inventors have shown that adding about 2 g of calcium lactate to a beverage with a pH of between 3.5 and 3.0 can sometimes result in an acid sensation or acid perception which is typically associated with beverages with a pH which is about 0.5 pH point lower. Incidentally, a trained taste panel is usually already capable of indicating an acid perception effect of 0.2-0.3 pH point. It is noted that there is no logical correlation between the acid sensation as perceived by a trained taste panel (member) and the actual value of the pH.

In a preferred aspect, the present invention is directed to a fruit juice containing beverage, and preferably fruit juice, with an acid sensation which is normally only obtained with such a beverage with a lower pH, which fruit juice comprises at least 0.5 gram per liter calcium lactate, most preferably between 1 and 5/gl calcium lactate.

Incidentally, in WO-98/32344, a document which focuses on fighting osteoporosis, a calcium-enriched beverage is described. This beverage has a low pH of 2.8-4.6. In example 1 of this document, a taste experiment is described in which the effect of different calcium sources on the taste acceptance is investigated. It does not follow from this experiment, where compositions are compared which, in addition to different calcium sources, also have different calcium concentrations, different amounts of sweetener and differences in pH, that calcium lactate has an acid sensation increasing effect.

The amount of lactate, and in particular calcium lactate, to be added depends on the composition of the beverage of which the acid perception needs to be increased. Milk and acid milk products, for instance, have a composition which is not erosive, particularly due to the calcium and phosphate contents present therein by nature; thus, little advantage can be gained by adding calcium lactate.

This is different for fruit juices, fruit lemonades, nectars, soft drinks and products based on dairy and fruit juices, such as whey-containing products having therein a high fruit juice content. These products are - depending on particularly pH, buffer capacity and calcium phosphate content - to a greater or lesser extent erosive to teeth. In these products, the increased acid perception by use of lactate ions and particularly calcium lactate can result in the pH of the beverage possibly being higher, so that the beverage as such acts has a less erosive effect on the teeth.

Incidentally, in this specification and the appended claims, the following terms are defined as follows, while the definitions are substantially based or to be based on Directive 2001/112/EC of the Council of December 20, 2001 relating to fruit juices and certain similar products intended for human consumption; from the European Food Legislation:
- fruit juices: the fermentable but unfermented product obtained from fruit which is sound and ripe, fresh or preserved by chilling, of one or more kinds mixed together, having the characteristic color, flavor and taste typical of the juice of the fruit from which it comes; flavor, pulp and cells from the juice which are separated during processing may be restored to the same juice.
- citrus fruit juice: fruit juice obtained from the endocarp, unless it is from limes; with lime juice, it may be obtained from the whole fruit;
- fruit juice from concentrate: the product obtained by replacing in the concentrated fruit juice water extracted from that juice during concentration, and restoring the flavors, and, if appropriate, pulp and cells lost from the juice but recovered during the process of producing the fruit juice in question or of fruit juice of the same kind; the water added must display appropriate characteristics, particularly from the chemical, microbiological and organoleptic viewpoints, in such a way as to guarantee the essential qualities of the juice;
- fruit nectar: the fermentable but unfermented product obtained by adding water and sugars and/or honey to fruit juice, concentrated fruit juice, hydrated fruit juice or fruit juice powder, to fruit puree or to a mixture of these products;
- lemonade and soft drink: a beverage which contains no alcohol, unless this is, due to a natural fermentation process, unintentionally and inevitably present to a content of at most 5 g of ethanol per liter, and which consists of water, natural mineral water or spring water; sugars or sweeteners; to which beverage, in any case, carbonic acid, aromas, edible components of fruits or plants, and/or fruit or plant juices may be added; and
- fruit lemonade: a lemonade with at least 10 wt.% fruit juice.

Beverages in which alcohol is present may also be made less erosive to the teeth utilizing the acid taste sensation increasing agent according to the invention.

In a dental erosion model based on (bovine) enamel blocks, it has been demonstrated that dental erosion is indeed reduced in compositions in which calcium lactate is included compared to compositions without calcium lactate with a lower pH, without those compositions containing calcium lactate having lost in respect of the taste attribute "acid"/"freshness".

This same dental erosion model can be used by a skilled person to set the amount of lactate ions, and particularly the amount of calcium lactate per orally applicable composition such that an optimal end product is obtained with regard to acid sensation and with regard to as little dental erosion-causing effect as possible.

In more detail, for the dental erosion model, freshly extracted bovine incisors, whose roots have been removed, are stored in water. The incisors are optically selected on the basis of absence of caries lesions or other visible defects. The enamel of the selected incisors is cut/sawn into rectangles of 3 by 3 millimeters, which rectangles are then embedded in polymethyl methacrylate PMMA, after which they are polished with ultrafine sandpaper (P600). These embedded enamel blocks are then placed in a test tube with 1 ml of the etching or non-etching composition to be tested therein. Agitation takes place at room temperature, for a predetermined time. After incubation, the embedded block is removed from the solution and patted dry and stored in distilled water. Both prior to and after the incubation, the etching or non-etching composition is subjected to a calcium analysis using atomic absorption spectroscopy in order to determine the amount of calcium leached out.

According to the invention, in the products according to the invention, preferably less than 0.1 ppm calcium per mm² of bovine incisor surface is leached out after 12 minutes of exposure.

In the following examples, a fruit juice according to the invention is described, and the results of a taste test are given.

**Example 1:**

| | |
|---|---|
| apple juice | 92 wt.% |
| white grape juice | 5 wt.% |
| Puracal-PP-FCC | 0.2 wt.% |
| water, aroma, vitamins | remainder |

### Example 2

In this example, by means of a ranking test, it is demonstrated that adding calcium lactate influences the acid perception in Dubbelfrisss® white grape lemon (DFWC).

To this end, 8 members of a specialized taste panel were offered the products, as described in the following Table 3, in duplicate so that a total of 16 measurements were generated. The products mentioned in Table 3 were offered in a randomized and coded manner. The panel members were to rank them according to the attribute "acid taste". Here, the most acid sample was to be put in first place, with 1 point being awarded. Later, the rankings were added up and it was determined how much difference is needed between the rank totals to be able to say that there are significant differences.

**TABLE 3**

| Product: | | |
|---|---|---|
| DFWC 100% calcium lactate | (1.81 g/l) | pH 3.44 |
| DFWC 0% calcium lactate | (0 g/l) | pH 3.17 |
| DFWC 50% calcium lactate | (0.905 g/1) | pH 3.32 |
| DFWC 200% calcium lactate | (3.621 g/l) | pH 3.12 |

From the test, the following results followed:

**TABLE 4**

| content of calcium lactate | | number of points | group |
|---|---|---|---|
| 0% | " | 48.00 | A |
| 50% | " | 44.00 | AB |
| 100% | " | 28.00 | BC |
| 200% | " | 20.00 | C |

These results lead to the following significant differences (products with the same letter fall within the same group and are not significantly different from one another):
0% and 50% calcium lactate are significantly less acid in taste than 200% calcium lactate; and
0% calcium lactate is significantly less acid in taste than 100% and 200% calcium lactate.

As a final conclusion, it can be stated that calcium lactate influences the acid perception of a product. The results indicate that, the more calcium lactate is present in the product, the more acid the product tastes.

## Claims

1. Use of a lactate ion source as an acid taste sensation increasing agent.

2. Use according to claim 1, wherein the lactate ion source comprises calcium ions.

3. Use according to claim 1 or 2, wherein the lactate ion source is calcium lactate.

4. Use according to any one of the preceding claims in a composition for oral application.

5. Use according to claim 4, wherein the composition for oral application is a food and particularly a beverage, preferably a fruit juice.

6. A method for increasing the acid sensation of an orally applicable composition comprising the addition of an effective amount of lactate ions to that composition.

7. A method according to claim 6, wherein the lactate ions are added in combination with calcium ions, most preferably in the form of calcium lactate.

8. A method according to claim 6 or 7, wherein the orally applicable composition to which the lactate ions are added is a food, such as a beverage and most preferably a fruit juice.

9. A method according to any one of claims 6-8, wherein the pH of the obtained composition is between 3 and 5.5.

10. A fruit juice containing beverage, and preferably fruit juice, comprising at least 0.5 gram per liter calcium lactate, most preferably between 1 and 5 g/l calcium lactate.
